# EUROPEAN PATENT SPECIFICATION

(11) **EP 2 873 419 B1**
(45) Date of publication and mention of the grant of the patent: **07.12.2016**
(21) Application number: 13816643.4
(22) Date of filing: 10.07.2013
(51) Int. Cl.: A61K 31/52, A61P 35/00, A61K 31/495

(54) **PHARMACEUTICAL COMPOSITION FOR THE PREVENTION OR TREATMENT OF BRAIN TUMOR OR TEMODAL-RESISTANT GLIOBLASTOMA MULTIFORM COMPRISING AZATHIOPRINE AS ACTIVE INGREDIENT**
PHARMAZEUTISCHE ZUSAMMENSETZUNG MIT AZATHIOPRIN ALS WIRKSTOFF ZUR PRÄVENTION BZW. BEHANDLUNG VON GEHIRNTUMOR ODER TEMODALRESISTENTEM GLIOBLASTOMA MULTIFORM
COMPOSITION PHARMACEUTIQUE COMPRENANT DE L'AZATHIOPRINE EN TANT QUE PRINCIPE ACTIF POUR LA PRÉVENTION OU LE TRAITEMENT DE TUMEUR DU CERVEAU OU DE GLIOBLASTOME MULTIFORME RÉSISTANT À TÉMODAL

(30) Priority: 11.07.2012 KR 20120075365
(43) Date of publication of application: 20.05.2015
(73) Proprietor: Korea Research Institute of Chemical Technology, Daejeon 305-343 (KR); Samsung Life Public Welfare Foundation, Seoul 140-893 (KR)
(72) Inventor: CHO, Heeyeong, Daejeon 305-761 (KR); CHOI, Gildon, Daejeon 305-751 (KR); PARK, Woo Kyu, Cheongju-si Chungcheongbuk-do 361-852 (KR); JEONG, Dae Young, Daejeon 305-340 (KR); KIM, Hyeon Young, Busan 607-010 (KR); LEE, Sunkyung, Daejeon 305-707 (KR); NAM, Do Hyun, Seoul 143-758 (KR); SEOL, Ho Jun, Seoul 137-906 (KR)
(74) Representative: Office Freylinger
(86) International application number: PCT/KR2013/006157
(87) International publication number: WO 2014/010948

(56) References cited:
- WO-A1-2011/017583
- US-A- 5 733 915
- GIANLUIGI ZAZA ET AL: "Thiopurine pathway", PHARMACOGENETICS AND GENOMICS, vol. 20, no. 9, 1 September 2010 (2010-09-01), pages 573-574, XP055186994, ISSN: 1744-6872, DOI: 10.1097/FPC.0b013e328334338f
- Y IWADATE ET AL: "Promising survival for patients with glioblastoma multiforme treated with individualised chemotherapy based on in vitro drug sensitivity testing", BRITISH JOURNAL OF CANCER, vol. 89, no. 10, 17 November 2003 (2003-11-17), pages 1896-1900, XP055186968, ISSN: 0007-0920, DOI: 10.1038/sj.bjc.6601376
- IWADATE: "Selection of chemotherapy for glioblastoma expressing O6-methylguanine-DNA methyltransferase", EXPERIMENTAL AND THERAPEUTIC MEDICINE, vol. 1, no. 1, 11 November 2009 (2009-11-11), XP055186965, ISSN: 1792-0981, DOI: 10.3892/etm_00000009
- XIANZHEN CHEN ET AL: "Bioinformatics analysis reveals potential candidate drugs for different subtypes of glioma", NEUROLOGICAL SCIENCES, vol. 34, no. 7, 30 September 2012 (2012-09-30), pages 1139-1143, XP055186909, ISSN: 1590-1874, DOI: 10.1007/s10072-012-1198-3
- MALTZMAN, JONAHAN S. ET AL.: 'Azathioprine: old drug, new actions' THE JOURNAL OF CLINICAL INVESTIGATION vol. 111, no. 8, 2003, pages 1122 - 1124, XP055172964
- PATEL, ACASH A. ET AL.: 'Azathioprine in dermatology: The past, the present, and the future' JOURNAL OF THE AMERICAN ACADEMY OF DERMATOLOGY vol. 55, no. 3, 2006, pages 369 - 389, XP005603404

## Description

### BACKGROUND OF THE INVENTION

### 1. Field of the Invention

The present invention relates to a pharmaceutical composition for use in the prevention or treatment of glioblastoma multiform or Temodal resistant glioblastoma multiform comprising azathioprine as an active ingredient.

### 2. Description of the Related Art

A tumor developed in glial cells providing nourishments to neurons is called glioma. Such glioma is histologically classified into 4 grades by considering atypia of nucleus, necrosis, vascular endothelial cell proliferation grade (most malignant grade), and mitotic property. When it is determined as grade 4, it is diagnosed as glioblastoma multiform (GBM) [Newlands E.S. et al. Eur. J. Cancer 32A, 22362241, 1996].

Glioblastoma multiform is the most malignant glioma and at the same time the most frequent brain tumor. It takes 25% of adult glioma and 15% of children glioma cases. There is no effective drug for the disease and only surgical operation and radiotherapy are performed. Prognosis of this disease is way worse than those of other cancers. The average survival period is only about 14 months [Chakravarti A. et al. Clin Cancer Res. 12, 47384746, 2007].

The recent studies on the expressions of a variety of genes suspected to be involved in glioblastoma multiform confirmed that there are 4 subtypes of glioblastoma multiform. The four subtypes of glioblastoma multiform are proneural subtype, mesenchymal subtype, classical subtype, and neural subtype. The survival period differs from the subtype and the response to the conventional treatment drugs also differs from the subtype. The average age of patients is also different from each subtype [H.S. Phillips H. S. et al., Cancer Cell, 9, 157173, 2006].

The gene over-expressed or mutated is also different among the subtypes of glioblastoma multiform [Cancer Genome Atlas Research Network, Nature, 455, 10611068, 2008]. In classical subtype, EGFR (epidermal growth factor receptor) is abnormally over-expressed. Unlike three other subtypes, the classical subtype does not display mutation in 'p53', the tumor suppressor gene. The treatment progress is comparatively good in this classical subtype patients, compared with other subtype patients. In proneural subtype, the mutation rate of 'p53' gene is high, and so is the mutation rate of IDH1 (isocitrate dehydrogenase) gene and PDGFRA (platelet-derived growth factor receptor-α) gene, unlike in classical subtype. The mutation in these genes plays an important role in cancer cell proliferation. The mutation of PDGFRA (platelet-derived growth factor receptor-α) gene is only observed in proneural subtype. The average age of proneural subtype patients is younger than other subtype patients' average age, but the survival period of this type cannot be extended by the current treatment method. In mesenchymal subtype, not only the mutation rate of 'p53' but also the mutation rates of NF1 and PTEN tumor suppressor genes are high. In neural subtype, the general gene mutation frequently observed in other subtypes is also observed, which is not particularly higher or lower than others.

The recent treatment method for glioblastoma multiform is mainly focused on the elimination of tumor by surgical operation together with radiotherapy and chemo-therapy using Temodal (temozolomide).

Since an efficient drug that can cure glioblastoma multiform has not been developed yet, Temodal is the only oral anticancer agent for glioblastoma multiform. When Temodal is administered to a patient, the cancer in the patient seems to response in the beginning. However, it shows resistance against the drug fast and once resistance is seen, the treatment effect is actually no more expected. Therefore, it is urgently requested to develop an efficient anticancer agent to cure brain tumor, particularly to treat Temodal-resistant tumor. As mentioned hereinbefore, 4 subtypes of glioblastoma multiform have different genetic backgrounds, so that a subtype specific drug has to be developed.

Temodal is self-degraded in cells to generate 5-(3-methyltriazine-1-yl)-imidazole-4-carboxamide (MITC). And MITC generates 5-aminoimidazole-4-carboximide, the active material. 5-Aminoimidazole-4-carboximide can methylate purine nucleotides of DNA, precisely N7 of guanine (70%), N3 of adenine (9%), and 06 of guanine (6%). In particular, the methylation of 06 of guanine is an important key of the toxicity of Temodal to cancer cells. The modified DNA nucleotide 06-methyl guanine induces discrepancy in between DNA strands during DNA duplication process, resulting in apoptosis [Newlands E.S, et al. Cancer Treat. Rev. 23, 3561, 1997].

In the meantime, the DNA repair protein MGMT (06-me-G methyltransferase) plays a role in eliminating the methyl residue from the methylated guanine 06. The protein activity of MGMT differs from cancer patients with displaying the biggest gap of 300 times the activity [Silber J. R. et al., Clin Cancer Res. 5, 807814, 1999]. When MGMT gene promoter is epigenetically methylated, the expression of MGMT gene is suppressed. This phenomenon is observed in about 45% of glioma patients [Hegi M. E. et al., N Engl J Med. 352, 9971003, 2005]. In those patients showing the suppression of MGMT gene expression by the methylation of MGMT gene promoter, Temodal worked well. On the other hand, in those patients showing the over-expression of MGMT, Temodal resistance was observed and as a result the treatment was frequently unsuccessful [Bobola M. S. et al., Clin Cancer Res. 2, 735741, 1996].

To overcome the resistance of alkylating agents by MGMT activity, it is often tried to inhibit MGMT protein activity by using a non-toxic fake substrate. As the fake substrate, 06-benzylguanine [Dolan M. E. et al., Proc Natl Acad Sci USA 87, 53685372, 1990] or [06-(4-bromothenyl)guanine] is used. These substrates deliver benzyl or bromobenzyl residue to cysteine of an enzyme to inactivate the enzyme [Clemons M. et al., Brit J Can 93, 11521156, 2005]. When 06-(4-bromothenyl)guanine is co-treated with DNA alkylating agents, the side effect (myelosuppression) is observed, because of which the use of it is limited [Gerson S. L. et al., J Clin Oncol 20, 23882399, 2002] . In addition to the activity of MGMT, the activity of MMR (mismatch repair) protein complex that can repair DNA strand mismatch is also closely related to Temodal resistance [Esteller M. and Herman J. G. Oncogene 23, 18, 2004]. As the activity of MMR protein complex increases, the recognition of Temodal mediated DNA modification by cells is so clear that DNA strand breakage and cell death are easily occurred. When the MMR activity is reduced by genetic factors or environmental factors, Temodal mediated DNA modification does not lead to cell death and rather Temodal resistance increases [Palombo F. et al., Science 268, 19121914, 1995].

Azathioprine is the drug to treat rheumatoid arthritis and kidney transplantation patients, which is recently administered to treat Crohn's disease [Sandborn, W. J. Scandinavian journal of gastroenterology. Supplement 225, 9299, 1998]. Azathioprine is a precursor-type drug, which is converted into 6-mercaptopurine (6-MP) in cells and then converted again into thioinosine monophosphate (TIMP) by hypoxanthine phosphoribosyl transferase (HPRT). The generated TIMP is converted into methylthioinosine nucleotide (meTIMP) by thiopurine methyltransferase (TPMT). The generated meTIMP acts as a purine synthesis inhibitor *in vivo.* In the meantime, 6-MP originated TIMP is converted into thioguanine nucleotide through another metabolic pathway, it is inserted instead of normal nucleotides in DNA or RNA biosynthesis to cause cytotoxicity [Tidd D. M. and Paterson A. R. Cancer Res 34: 738746, 1974].

Thioguanine nucleotide generated by azathiopurine is a different DNA modification distinguished in its structure from guanine 06-methyl generated by Temodal. Therefore, it is assumed that this modification is not affected by the activity of MGMT gene that is a reason of Temodal resistance. So, it has the mechanism useful for the treatment of glioblastoma multiform patients showing Temodal resistance.

Y. IWADATE et al. in "Promising survival for patients with glioblastoma multiforme treated with individualised chemotherapy based on in vitro drug sensitivity testing", Brit J CAN, vol. 89, no. 10, 17 November 2003, pages 1896-1900, present a retrospective investigation on in vitro drug sensitivity testing of 30 anticancer agents on a total of 40 patients with GBM based on in vitro measurement of apoptosis. Furthermore, Y. IWADATE et al. in "Selection of chemotherapy for glioblastoma expressing O6-methylguanine-DNA methyltransferase", EXP. AND THER. MEDICINE, vol.1, 11 November 2009, present a study of 25 anticancer agents on 74 patients with MGMT-positive GBM. Both studies conclude that individualized chemotherapy is a promising strategy for potentially prolonging the survival of GBM patients without however indicating particularly advantageous compounds.

In the course of study to find out an alternative compound that can take the place of Temodal, the conventional therapeutic agent for glioblastoma multiform, the present inventors confirmed that azathioprine could inhibit the proliferation of glioblastoma multiform, a kind of brain tumor, and was effective in treating glioblastoma multiform tumor cells particularly showing Temodal resistance, leading to the completion of this invention.

### SUMMARY OF THE INVENTION

It is an object of the present invention to provide a pharmaceutical composition for use in the prevention or treatment of glioblastoma multiform, comprising azathioprine or the pharmaceutically acceptable salts thereof as an active ingredient.

It is another object of the present invention to provide a pharmaceutical composition for use in the prevention or treatment of Temodal resistant glioblastoma multiform comprising azathioprine or the pharmaceutically acceptable salts thereof as an active ingredient.

It is also an object of the present invention to provide a compound or the pharmaceutically acceptable salts thereof for use in the prevention or treatment of glioblastoma multiform.

It is further an object of the present invention to provide a compound or the pharmaceutically acceptable salts thereof for use in the prevention or treatment of Temodal resistant glioblastoma multiform.

To achieve the above objects, the present invention provides a pharmaceutical composition for use in the prevention or treatment of glioblastoma multiform, comprising the compound represented by formula 1 or the pharmaceutically acceptable salts thereof as an active ingredient.

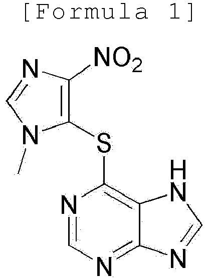

The present invention also provides a pharmaceutical composition for use in the prevention or treatment of Temodal resistant glioblastoma multiform comprising the compound represented by formula 1 or the pharmaceutically acceptable salts thereof as an active ingredient.

### ADVANTAGEOUS EFFECT

The azathioprine in the present invention is not only effective in inhibiting the growth of glioblastoma multiform, a kind of brain tumor, but also excellent in treating glioblastoma multiform that displays resistance against Temodal (temozolomide), the conventional therapeutic agent for glioblastoma multiform, so that it can be effectively used as an active ingredient of a pharmaceutical composition for use in the prevention or treatment of glioblastoma multiform and particularly Temodal resistant glioblastoma multiform.

### BRIEF DESCRIPTION OF THE DRAWINGS

The application of the preferred embodiments of the present invention is best understood with reference to the accompanying drawings, wherein:
Figure 1 is a graph illustrating the concentration of the azathioprine represented by formula 1 in concentration-response curve that is effective in inhibiting the cell proliferation. (In Figure 1, '559T' indicates proneural subtype glioblastoma multiform, '592T' indicates mesenchymal subtype glioblastoma multiform, '626T' indicates classical subtype glioblastoma multiform, 'NHA' indicates normal astrocytes, 'U87MG' indicates glioblastoma multiform cell line, and 'A549' indicates lung cancer cell line).
Figure 2 is a diagram illustrating the inhibition of neurosphere formation in glioblastoma multiform cells by the compound of Comparative Example 1 (Temodal).
Figure 3 is a diagram illustrating the inhibition of neurosphere formation in glioblastoma multiform cells by the compound of formula 1 in the present invention (azathioprine).
Figure 4 is a graph illustrating the survival rate and the survival period when the azathioprine in the present invention is co-treated with Temodal or radiotherapy for the treatment of glioblastoma multiform.

### DESCRIPTION OF THE PREFERRED EMBODIMENTS

Hereinafter, the present invention is described in detail.

The present invention relates to the use of a compound capable of inhibiting the growth of glioblastoma multiform (GBM), one of the malignant brain tumors. Particularly, the object of the present invention is to use the conventional azathioprine used to treat rheumatoid arthritis and kidney transplantation patients as a novel therapeutic agent for glioblastoma multiform, a kind of brain tumor, more specifically for the conventional glioblastoma multiform treating drug Temodal resistant glioblastoma multiform.

The present invention provides a pharmaceutical composition for use in the prevention or treatment of glioblastoma multiform comprising the compound represented by formula 1 or the pharmaceutically acceptable salts thereof as an active ingredient:

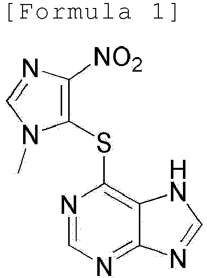

The pharmaceutical composition for use of the present invention can be applied to proneural subtype glioblastoma multiform, mesenchymal subtype glioblastoma multiform, classical subtype glioblastoma multiform, and neural subtype glioblastoma multiform, and to other non-classified glioblastoma multiform. It is more preferred to apply the pharmaceutical composition of the invention to mesenchymal subtype glioblastoma multiform and classical subtype glioblastoma multiform, but not always limited thereto.

When the compound used in the present invention (azathioprine) was treated to proneural subtype, mesenchymal subtype, and classical subtype glioblastoma multiform cancer cells originated from patients, and the general glioblastoma multiform cell line, and lung cancer cell line, the cancer cell growth inhibiting effect was observed. In the meantime, the compound did not show toxicity to normal astrocytes (see Experimental Example 1).

As described hereinbefore, the azathioprine used in the invention inhibits efficiently the growth of glioblastoma multiform, a kind of brain tumor. Thus, the compound use in the present invention can be effectively used as an active ingredient of a pharmaceutical composition for use in the prevention or treatment of glioblastoma multiform.

The present invention also provides a pharmaceutical composition for use in the prevention or treatment of Temodal resistant glioblastoma multiform comprising the compound represented by formula 1 or the pharmaceutically acceptable salts thereof as an active ingredient:

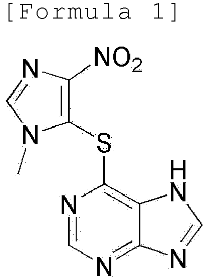

The recent treatment method for glioblastoma multiform is mainly focused on the elimination of tumor by surgical operation together with radiotherapy and chemo-therapy using Temodal (temozolomide). When Temodal is administered to a patient, the cancer in the patient seems to respond in the beginning. However, it shows resistance against the drug fast and once resistance is seen, the treatment effect is actually no more expected.

Therefore, it is urgently requested to develop an efficient anticancer agent to cure brain tumor (particularly, glioblastoma multiform), especially to treat Temodal resistant tumor.

The compound used in the present invention (azathioprine) was tested in 10 types of glioblastoma multiform. Among them, the treatment effect was weak only in GBM#1. The compound strongly inhibited neurosphere formation in the remaining 9 groups of glioblastoma multiform. In the meantime, even in GBM#1 showing weak response to azathioprine at the concentration of 1 µM, when the concentration of the compound was increased to 10 µM, the neurosphere formation was significantly inhibited, suggesting that azathioprine has a strong activity to every kind of glioblastoma multiform tested herein. The compound used in the present invention (azathioprine) demonstrated the anticancer effect by inhibiting neurosphere formation particularly in the glioblastoma multiform having resistance against the compound of Comparative Example 1 (Temodal). The above result indicates that the compound used in the present invention (azathioprine) has a wide spectrum of anticancer activity to glioblastoma multiform cancer cells, compared with the compound of Comparative Example 1 (Temodal). The confirmed activity to inhibit neurosphere formation even in Temodal resistant cancer cells makes the compound as an effective drug candidate to treat Temodal resistant patients (see Experimental Example 2).

The pharmaceutical composition comprising the azathioprine for use of the present invention as an active ingredient can be treated alone or co-treated with Temodal (temozolomide) to a subject having glioblastoma multiform.

As described hereinbefore, the azathioprine used in the present invention shows excellent treating effect on such glioblastoma multiform cancer cells exhibiting resistance against Temodal (temozolomide), the conventional glioblastoma multiform treating agent, so that it can be effectively used as an active ingredient of a pharmaceutical composition for use in the prevention or treatment of Temodal resistant glioblastoma multiform.

The composition comprising the compound represented by formula 1 for use of the present invention preferably includes the compound at the concentration of 0.1 ~ 50 weight% by the total weight of the composition, but not always limited thereto.

The composition for use of the present invention can additionally include generally used carriers, excipients and diluents.

The composition for use of the present invention can be formulated for oral administration, for example powders, granules, tablets, capsules, suspensions, emulsions, syrups and aerosols, and for parenteral administration, for example external use, suppositories and sterile injections, etc. Possible suitable formulations are oral preparations such as powders, granules, tablets, capsules, suspensions, emulsions, syrups and aerosols, preparations for external use, suppositories and sterile injections. The carriers, excipients and diluents are exemplified by lactose, dextrose, sucrose, sorbitol, mannitol, xylitol, erythritol, maltitol, starch, acacia rubber, alginate, gelatin, calcium phosphate, calcium silcate, cellulose, methyl cellulose, microcrystalline cellulose, polyvinyl pyrrolidone, water, methylhydroxybenzoate, propylhydroxybenzoate, talc, magnesium stearate and mineral oil.

Formulations can be prepared by using generally used excipients or diluents such as fillers, extenders, binders, wetting agents, disintegrating agents and surfactant. Solid formulations for oral administration are tablets, pills, powders, granules and capsules. These solid formulations are prepared by mixing one or more suitable excipients such as starch, calcium carbonate, sucrose or lactose, gelatin, etc. Except for the simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, emulsions, lyophilized preparations, suppositories and injections. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

The composition for use of the present invention can be administered orally or parenterally.

The effective dosage of the composition of the present invention can be determined according to weight and condition of a patient, severity of a disease, preparation of a drug, administration pathway and time.

The composition for use of the present invention can be administered alone or treated together with surgical operation, radiotherapy, hormone therapy, chemo-therapy and biological regulators.

The present invention also provides the compound represented by formula 1 or the pharmaceutically acceptable salts thereof for use in the prevention or treatment of glioblastoma multiform:

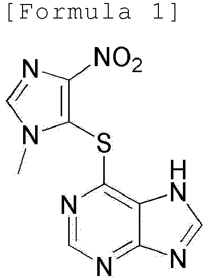

The compound represented by formula 1 used in the present invention can be applied to proneural subtype glioblastoma multiform, mesenchymal subtype glioblastoma multiform, classical subtype glioblastoma multiform, and neural subtype glioblastoma multiform, and to other non-classified glioblastoma multiform. It is more preferred to apply the pharmaceutical composition of the invention to mesenchymal subtype glioblastoma multiform and classical subtype glioblastoma multiform, but not always limited thereto.

In addition, the present invention provides the compound represented by formula 1 or the pharmaceutically acceptable salts thereof for use in the prevention or treatment of Temodal (temozolomide) resistant glioblastoma multiform.

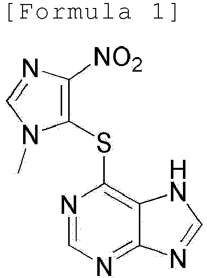

The compound represented by formula 1 can be treated alone or co-treated with Temodal (temozolomide) to a subject having glioblastoma multiform.

The compound represented by formula 1 can be used as the form of a pharmaceutically acceptable salt, in which the salt is preferably acid addition salt formed by pharmaceutically acceptable free acids. The acid addition salt can be obtained from inorganic acids such as hydrochloric acid, nitric acid, phosphoric acid, sulfuric acid, hydrobromic acid, hydroiodic acid, nitrous acid and phosphorous acid, or non-toxic organic acids such as aliphatic mono/dicarboxylate, phenyl-substituted alkanoate, hydroxy alkanoate, alkandioate, aromatic acids and aliphatic/aromatic sulfonic acids. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate and mandelate.

The acid addition salt in this invention can be prepared by the conventional method known to those in the art. For example, the compound of formula 1 is dissolved in an organic solvent such as methanol, ethanol, acetone, methylenechloride, or acetonitrile, to which organic acid or inorganic acid is added to induce precipitation. Then, the precipitate is filtered and dried to give the salt. Or the solvent and the excessive acid are distillated under reduced pressure, and dried to give the salt. Or the precipitate is crystallized in the organic solvent to give the same.

A pharmaceutically acceptable metal salt can be prepared by using a base. Alkali metal or alkali earth metal salt is obtained by the following processes: dissolving the compound in excessive alkali metal hydroxide or alkali earth metal hydroxide solution; filtering non-soluble compound salt; evaporating the remaining solution and drying thereof. At this time, the metal salt is preferably prepared in the pharmaceutically suitable form of sodium, potassium, or calcium salt. And the corresponding silver salt is prepared by the reaction of alkali metal or alkali earth metal salt with proper silver salt (ex; silver nitrate).

The present invention includes not only the compound represented by formula 1 but also the pharmaceutically acceptable salts thereof, solvates, and hydrates possibly produced from the same.

Further, the present document describes a method for treating cancer or Temodal (temozolomide) resistant glioblastoma multiform characterized by administering a pharmaceutical composition comprising the compound represented by formula 1 or the pharmaceutically acceptable salts thereof as an active ingredient to a subject having cancer or Temodal resistant glioblastoma multiform.

The pharmaceutical composition of the present invention is for use in the treatment of glioblastoma multiform.

The pharmaceutical composition for use of the present invention can be applied to proneural subtype glioblastoma multiform, mesenchymal subtype glioblastoma multiform, classical subtype glioblastoma multiform, and neural subtype glioblastoma multiform, and to other non-classified glioblastoma multiform. It is more preferred to apply the pharmaceutical composition of the invention to mesenchymal subtype glioblastoma multiform or classical subtype glioblastoma multiform, but not always limited thereto.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Preparation of 6-(1-methyl-4-nitro-1H-imidazol-5-ylthio)-7H-purine (azathioprine)

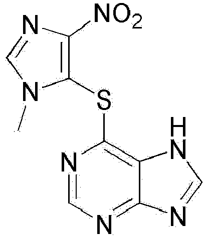

Azathioprine was purchased from Sigma Aldrich (product #: T2577) and used herein without any additional purification.

### Comparative Example 1: Preparation of Temodal (temozolomide)

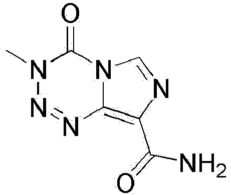

Temodal was purchased from Sigma Aldrich (product #: A4638) and used herein without any additional purification.

### Example 1: Evaluation of cell growth inhibition effect

Following experiment was performed to investigate the inhibitory effect of the compound represented by formula 1 used in the present invention (azathioprine) on glioblastoma multiform cancer cell growth.

Particularly, the cell growth inhibition effect was measured by WST-1 cell proliferation assay [http://www.roche-applied-science.com/PROD_INF/MANUALS/CELL_MAN/apoptosis_0 87_08 8.pdf]. The compound of formula 1 was loaded in a 384-well cell culture plate. Three types of glioblastoma multiform cell lines [proneural subtype (559T), mesenchymal subtype (592T), and classical subtype (626T)], normal human astrocytes (NHA), a lung cancer cell line (A549), and a glioblastoma multiform cell line (U87MG) were distributed in the plate at the density of 4.5 x 10³ cells/well, and the medium was filled up to make 50 *µ*ℓ*,* per well. At this time, the concentration of the solvent DMSO for the compound of formula 1 was no more than 0.2%. After culturing the cells for 72 hours, 5 mg/ml of WST-1 reagent was added by 5 *µ*ℓ*,* per well, followed by reaction for 3 ~ 4 hours. Then OD₄₅₀ was measured to investigate the effect of the compound of formula 1 on cell proliferation.

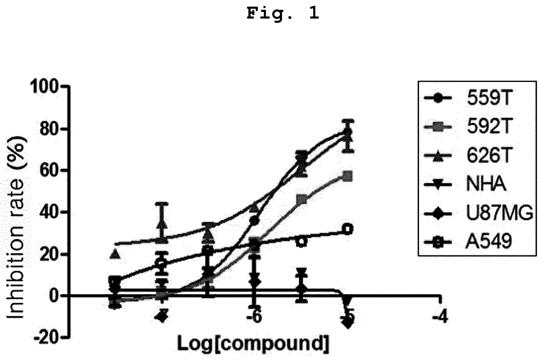

The results are presented in Table 1 and Figure 1.

**[Table 1]**

| Cell Line | Cell Growth Inhibition (% Inhibition) | | | | | | GI₅₀ (µM) |
|---|---|---|---|---|---|---|---|
| | 10 µM | 3.2 µM | 1.0 µM | 0.32 µM | 0.1 µM | 0.032 µM | |
| 559T | 78.6 | 65.8 | 35.9 | 9.7 | 2 . 6 | -4.2 | 1.3 |
| 592T | 57.2 | 46.0 | 22.6 | 8 . 3 | 2 . 1 | -3.9 | 5.5 |
| 626T | 76.3 | 61.8 | 42.5 | 30.0 | 34.9 | 20.2 | 3.1 |
| NHA | -2.4 | 10.5 | 8 . 3 | 11.0 | 5 . 9 | 1.9 | > 10 |
| U87MG | -12.7 | 3.2 | 6.5 | 10.5 | -9.9 | 3.2 | > 10 |
| A549 | 32.0 | 26.0 | 24.2 | 21.5 | 15.3 | 6.7 | > 10 |

Figure 1 is a graph illustrating the concentration of the azathioprine represented by formula 1 used in the present invention in concentration-response curve that is effective in inhibiting the cell proliferation. (In Figure 1, '559T' indicates proneural subtype glioblastoma multiform, '592T' indicates mesenchymal subtype glioblastoma multiform, '626T' indicates classical subtype glioblastoma multiform, 'NHA' indicates normal astrocytes, 'U87MG' indicates glioblastoma multiform cell line, and 'A549' indicates lung cancer cell line).

As shown in Table 1 and Figure 1, when the compound of formula 1 (azathioprine) was treated to proneural (559T), mesenchymal (592T) and classical (626T) subtype glioblastoma multiform cancer cells, the 50% growth inhibition concentration (GI₅₀) was respectively 1.3, 5.5, and 3.1 µM, indicating the cell growth inhibition effect was greatest in proneural subtype glioblastoma multiform. In the meantime, the compound did not cause toxicity in normal astrocytes. The cell growth inhibition effect of the compound was still observed in lung cancer cell line (A549) even though it was not as strong as the above. U87MG is a kind of glioblastoma multiform cancer cell line, but is a modified cell line for *in vitro* experiment. Azathioprine did not inhibit the cancer cell growth in this cell line.

Therefore, it was confirmed that the compound of formula 1 used in the present invention was excellent in inhibiting the growth of glioblastoma multiform, in particular, in inhibiting the growth of proneural subtype glioblastoma multiform, among many subtypes of glioblastoma multiform, suggesting that it can be effectively used as an active ingredient of a pharmaceutical composition for the prevention or treatment of cancer including brain tumor (particularly, glioblastoma multiform).

### Example 2: Evaluation of neurosphere formation inhibition effect

The inhibition of neurosphere formation in glioblastoma multiform cells was examined in order to investigate the anticancer effect of the compound of formula 1 (azathioprine) and the compound of Comparative Example 1 (Temodal).

Particularly, to investigate neurosphere formation, glioblastoma multiform cells were cultured for a certain time and the cells were distributed again in culture vessels, followed by additional culture. Then, the number of newly generated neurospheres was measured. First, the glioblastoma multiform patient originated cells were sub-cultured, which were distributed in a 96-well plate (20, 50, and 200 cells in 50 *µl,* of culture medium/well). 2, 6, and 20 µM of the compound of formula 1 (azathioprine) and the compound of Comparative Example 1 (Temodal) were treated thereto by 50 *µ*ℓ*,* per well to make the final concentrations of 1, 3, and 10 µM. Then, the compound of formula 1 (azathioprine) and the compound of Comparative Example 1 (Temodal) were added to 50 *µ*ℓ*,* of cell culture medium with the same concentrations as the above once a week, which were then cultured for 7 ~ 23 days. When neurosphere was formed at least 50 *µ*m in the control group not treated with the compound, the number of neurospheres in each well was counted to compare the neurosphere formation before and after the treatment of the compound of formula 1 (azathioprine) and the compound of Comparative Example 1 (Temodal).

The inhibition of neurosphere formation was observed in 10 kinds of glioblastoma multiform cells originated from glioblastoma multiform patients. The glioblastoma multiform cells used in this experiment were classified into 4 types, which are type A, type B, type C, and type D, according to the gene expression pattern. Methylation of MGMT (06-me-G methyltransferase), the DNA repair protein, was confirmed in each cell line. At that time, type A [GBM #1, #2, #3] was proneural subtype, type B [GBM #4, #5, #6, #7] was classical subtype, type C [GBM #8] was mesenchymal subtype, and type D [GBM #9, #10] was non-classified glioblastoma multiform.

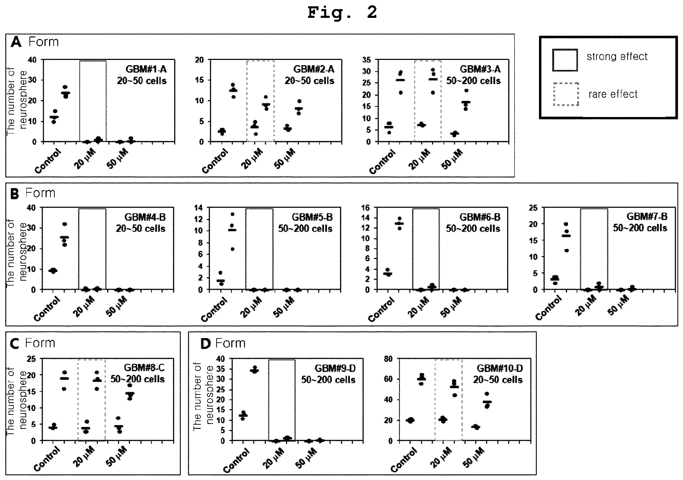

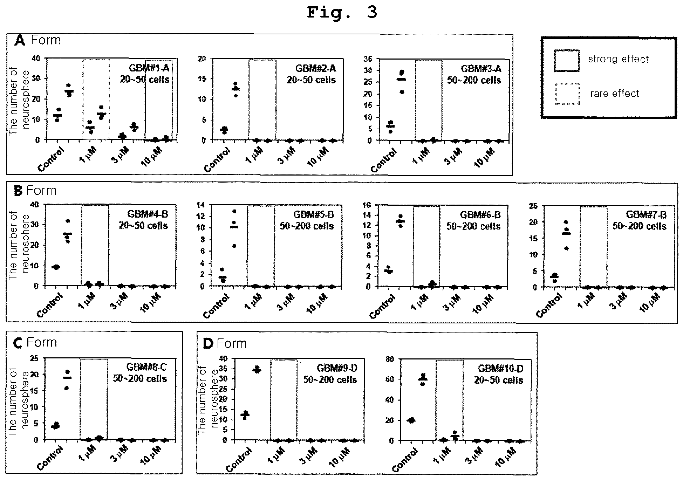

The results are presented in Table 2 and Figures 2 and 3.

**[Table 2]**

| Cell Line | Type | Duration (day) | Temodal | Azathiopr ine | MGMT Gene |
|---|---|---|---|---|---|
| | | | 20 µM | 1 µM | |
| GBM #1 | A | 7 | S | R | M |
| GBM #2 | A | 15 | R | S | UM |
| GBM #3 | A | 21 | R | S | UM |
| GBM #4 | B | 9 | S | S | M/UM |
| GBM #5 | B | 21 | S | S | M < UM |
| GBM #6 | B | 23 | S | S | M > UM |
| GBM #7 | B | 23 | S | S | ** |
| GBM #8 | C | 15 | R | S | UM |
| GBM #9 | D | 21 | S | S | M > UM |
| GBM #10 | D | 10 | R | S | UM |

| | | | | | |
|---|---|---|---|---|---|
| A: proneural subtype, B: classical subtype, C: mesenchymal subtype, D: non-classified glioblastoma multiform, S: strong effect, I: intermediate effect, R: rare effect, M: gene promoter methylated, UM: gene promoter unmethylated, and **: unvalued. | | | | | |

Figure 2 is a diagram illustrating the inhibition of neurosphere formation in glioblastoma multiform cells by the compound of Comparative Example 1 (Temodal).

Figure 3 is a diagram illustrating the inhibition of neurosphere formation in glioblastoma multiform cells by the compound of formula 1 used in the present invention (azathioprine).

As shown in Table 2 and Figures 2 ∼ 3, the compound of Comparative Example 1 (Temodal) could not inhibit neurosphere formation in the glioblastoma multiform suspected to show high MGMT activity because the gene promoter was not methylated (This glioblastoma multiform is presented as "UM" in Table 2). However, the compound of Comparative Example 1 significantly inhibited neurosphere formation in the glioblastoma multiform cells showing high level of MGMT gene methylation.

The compound of formula 1 (azathioprine) significantly inhibited neurosphere formation in 9 out of 10 tested glioblastoma multiform subtypes, except in GBM#1 which only showed a minor inhibitory effect. Even in GBM#1 showing a minor inhibitory effect with 1 µM of azathioprine, the neurosphere formation was significantly inhibited when the concentration of azathioprine was raised to 10 µM. From the above result, it can be judged that the inhibitory effect of the compound of formula 1 on neurosphere formation was all very strong in every tested glioblastoma multiform subtypes.

In particular, the compound of formula 1 (azathioprine) demonstrated anticancer effect by inhibiting neurosphere formation even in such glioblastoma multiform cancer cells that show a strong resistance against the compound of Comparative Example 1 (Temodal), which were GBM#2, #3, #8, and #10. So, the results above confirmed that the compound of formula 1 (azathioprine) has a wider spectrum of anticancer effect on glioblastoma multiform cancer cells than the compound of Comparative Example 1 (Temodal) and is even able to inhibit neurosphere formation in Temodal resistant cancer cells, providing a potential of the compound as an effective therapeutic agent for Temodal resistant cancer patients.

The compound of formula 1 (azathioprine) used in the present invention showed a treatment effect on a wide range of glioblastoma multiform cancer cells and on such glioblastoma multiform cancer cells that display resistance against the compound of Comparative Example 1 (Temodal), so that it can be effectively used as an active ingredient of a pharmaceutical composition for the prevention or treatment of cancer including brain tumor (particularly glioblastoma multiform) and as an active ingredient of a pharmaceutical composition for the prevention or treatment of Temodal (temozolomide) resistant glioblastoma multiform.

### Example 3: Animal test for evaluation of therapeutic effect using Temodal resistant cells

Following experiment was performed to investigate the radiosensitizing effect and chemosensitizing effect of the compound of formula 1 (azathioprine) in the glioblastoma multiform orthotopic animal model.

Particularly, to investigate the resistance against Temodal or radiotherapy in glioblastoma multiform, glioblastoma multiform cells were cultured. The cultured cells (2 x 10⁵/5*µ*ℓ) were transplanted in the brain of a mouse. 1 7 days later, the administration of Temodal (65 mg/kg, oral-administration) or radiotherapy (2 Gy) began, which continued for 5 days. The average survival period was investigated. The survival period extension rate (%) of each radiotherapy group and Temodal administration group was calculated by considering the survival period of the control that had not been treated with radiotherapy or Temodal as a standard. The results are presented in Table 3. For the comparison, the survival period of the group treated with "Avastin", the well-known anticancer agent, is also presented.

**[Table 3]**

| Experimental Group | Average Survival Period (day) | Survival Period Extension Rate (%) |
|---|---|---|
| Control | 27 | |
| Radiotherapy Group | 28 | 8 |
| Temodal Treated Group | 31 | 19 |
| Avastin Treated Group | 28 | 8 |

As shown in Table 3, the average survival period was not much extended in those mice transplanted with glioblastoma multiform cells and treated with radiotherapy or Temodal independently because of the resistance.

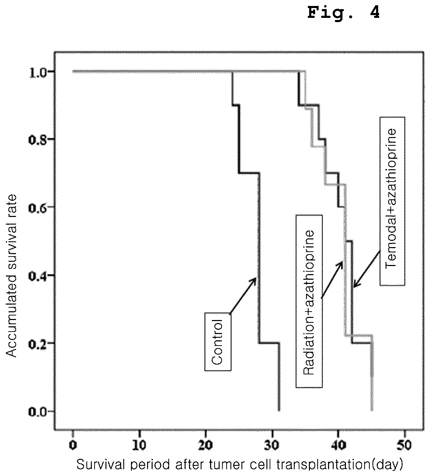

Glioblastoma multiform, glioblastoma multiform cells were cultured. The cultured cells (2 x 10⁵/5*µ*ℓ) were transplanted in the brain of a mouse. 17 days later, the administration of Temodal (65 mg/kg, oral-administration) or radiotherapy (2 Gy) began, which continued for 5 days. Unlike the above experiment, the compound of formula 1 used in the present invention (azathioprine) was co-treated to the mouse by oral-administration at the dose of 20 mg/kg every day after 11 days from the glioblastoma multiform transplantation in the mouse brain. Then, the average survival period was investigated. The survival period extension rate (%) of each Temodal+azathioprine administration group and radiotherapy+azathioprine treatment group was calculated by considering the survival period of the control that had not been treated with radiotherapy or Temodal as a standard. The results are presented in Table 4 and Figure 4.

**[Table 4]**

| Experimental Group | Average Survival Period (day) | Survival Period Extension Rate (%) | P value (vs. control) |
|---|---|---|---|
| Control | 28 | | |
| Temodal+azath ioprine Administratio n group | 41 | 43 | 0.000 |
| Radiotherapy +azathioprine treatment group | 41 | 44 | 0.000 |

Figure 4 is a graph illustrating the survival rate and the survival period when the azathioprine used in the present invention is co-treated with Temodal or radiotherapy for the treatment of glioblastoma multiform.

As shown in Table 4 and Figure 4, when the azathioprine used in the present invention was co-treated with Temodal or radiotherapy for the treatment of glioblastoma multiform, the survival period was significantly extended. This result indicates that the azathioprine used in the present invention can reduce the resistance against Temodal or radiotherapy in the treatment of glioblastoma multiform.

Therefore, it was confirmed that the compound of formula 1 used in the present invention (azathioprine) had the treatment effect on glioblastoma multiform cancer cells showing resistance against Temodal and/or radiotherapy, so that it can be effectively used as an active ingredient of a pharmaceutical composition for the prevention or treatment of cancer including brain tumor (particularly, glioblastoma multiform), and also can be effectively used as an active ingredient of a pharmaceutical composition for the prevention or treatment of Temodal resistant glioblastoma multiform.

The compound represented by formula 1 used in the present invention can be formulated in many forms according to the purpose of use. Here are the examples of the formulation comprising the compound represented by formula 1 of the invention as an active ingredient, but not always limited thereto.

### Manufacturing Example 1: Preparation of pharmaceutical formulations

### <1-1> Preparation of powders

| | |
|---|---|
| Compound of formula 1 | 500 mg |
| Lactose | 10 mg |
| Talc | 10 mg |

Powders were prepared by mixing all the above components, which were filled in airtight packs according to the conventional method for preparing powders.

### <1-2> Preparation of tablets

| | |
|---|---|
| Compound of formula 1 | 500 mg |
| Corn starch | 10 mg |
| Lactose | 10 mg |
| Magnesium stearate | 2 mg |

Tablets were prepared by mixing all the above components by the conventional method for preparing tablets.

### <1-3> Preparation of capsules

| | |
|---|---|
| Compound of formula 1 | 500 mg |
| Corn starch | 10 mg |
| Lactose | 10 mg |
| Magnesium stearate | 2 mg |

Capsules were prepared by mixing all the above components, which were filled in gelatin capsules according to the conventional method for preparing capsules.

### <1-4> Preparation of injectable solutions

| | |
|---|---|
| Compound of formula 1 | 500 mg |
| Sterilized distilled water | proper amount |
| pH regulator | proper amount |

Injectable solutions were prepared by mixing all the above components, putting the mixture into 2 mℓ ampoules and sterilizing thereof by the conventional method for preparing injectable solutions.

### <1-5> Preparation of liquid formulations

| | |
|---|---|
| Compound of formula 1 | 10 mg |
| Isomerized sugar | 10 g |
| Mannitol | 5 g |
| Purified water | proper amount |

All the above components were dissolved in purified water. After adding lemon flavor, total volume was adjusted to be 100 mℓ by adding purified water. Liquid formulations were prepared by putting the mixture into brown bottles and sterilizing thereof by the conventional method for preparing liquid formulations.

### INDUSTRIAL APPLICABILITY

The azathioprine used in the present invention is not only effective in inhibiting the growth of glioblastoma multiform, a kind of brain tumor, but also excellent in treating glioblastoma multiform that displays resistance against Temodal (temozolomide), the conventional therapeutic agent for glioblastoma multiform, so that it can be effectively used as an active ingredient of a pharmaceutical composition for the prevention or treatment of such cancer as brain tumor (particularly, glioblastoma multiform) and particularly Temodal resistant glioblastoma multiform.

## Claims

1. A pharmaceutical composition for use in preventing or treating glioblastoma multiform comprising the compound represented by formula 1 or the pharmaceutically acceptable salts thereof as an active ingredient:

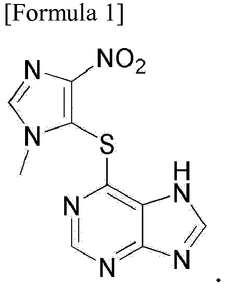

2. The pharmaceutical composition for use according to claim 1, wherein the glioblastoma multiform has the subtypes of proneural subtype, mesenchymal subtype, classical subtype, and neural subtype.

3. A pharmaceutical composition for use in preventing or treating Temodal (temozolomide) resistant glioblastoma multiform comprising the compound represented formula 1 or the pharmaceutically acceptable salts thereof as an active ingredient:

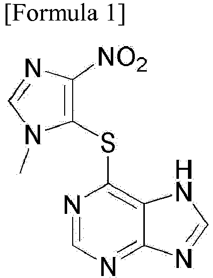

4. The pharmaceutical composition for use according to claim 3, wherein the pharmaceutical composition is administered alone or together with Temodal (temozolomide).

5. The pharmaceutical composition for use according to claim 3, wherein the glioblastoma multiform has the subtypes of proneural subtype, mesenchymal subtype, classical subtype, and neural subtype.

## Patentansprüche

1. Pharmazeutische Zusammensetzung zur Verwendung beim Verhindern oder Behandeln von Glioblastoma multiforme, wobei die Zusammensetzung die durch die Formel 1 dargestellte Verbindung oder die pharmazeutisch akzeptablen Salze davon als einen Wirkbestandteil umfasst:

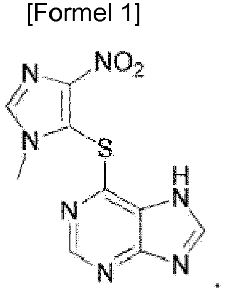

2. Pharmazeutische Zusammensetzung zur Verwendung nach Anspruch 1, wobei das Glioblastoma multiforme die Subtypen des proneuralen Subtyps, mesenchymalen Subtyps, klassischen Subtyps und neuralen Subtyps aufweist.

3. Pharmazeutische Zusammensetzung zur Verwendung beim Verhindern oder Behandeln von Temodal-resistentem (Temozolomid-resistentem) Glioblastoma multiforme, wobei die Zusammensetzung die durch die Formel 1 dargestellte Verbindung oder die pharmazeutisch akzeptablen Salze davon als einen Wirkbestandteil umfasst:

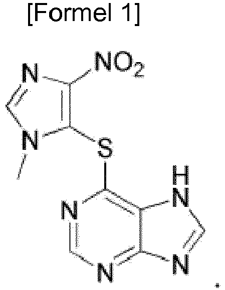

4. Pharmazeutische Zusammensetzung zur Verwendung nach Anspruch 3, wobei die pharmazeutische Zusammensetzung allein oder zusammen mit Temodal (Temozolomid) verabreicht wird.

5. Pharmazeutische Zusammensetzung zur Verwendung nach Anspruch 3, wobei das Glioblastoma multiforme die Subtypen des proneuralen Subtyps, mesenchymalen Subtyps, klassischen Subtyps und neuralen Subtyps aufweist.

## Revendications

1. Composition pharmaceutique pour son utilisation dans la prévention ou le traitement du glioblastome multiforme comprenant le composé répondant à la formule 1 ou ses sels pharmaceutiquement acceptables à titre d'ingrédient actif :

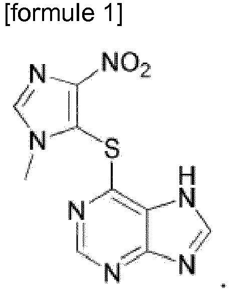

2. Composition pharmaceutique pour son utilisation selon la revendication 1, dans laquelle le glioblastome multiforme possède comme sous-types, le sous-type proneural, le sous-type mésenchymateux, le sous-type classique et le sous-type neuronal.

3. Composition pharmaceutique pour son utilisation dans la prévention ou le traitement du glioblastome multiforme résistant au témodal (témozolomide) comprenant le composé répondant à la formule 1 ou ses sels pharmaceutiquement acceptables à titre d'ingrédient actif :

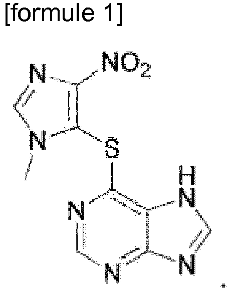

4. Composition pharmaceutique pour son utilisation selon la revendication 3, dans laquelle la composition pharmaceutique est administrée seule ou conjointement avec du témodal (témozolomide).

5. Composition pharmaceutique pour son utilisation selon la revendication 3, dans laquelle le glioblastome multiforme possède comme sous-types, le sous-type proneural, le sous-type mésenchymateux, le sous-type classique et le sous-type neuronal.
